# EUROPEAN PATENT APPLICATION

(11) **EP 1 767 217 A1**
(43) Date of publication of application: **28.03.2007**
(21) Application number: 05765630.8
(22) Date of filing: 13.07.2005
(51) Int. Cl.: A61K 45/00, A61K 31/137, A61K 31/165, A61K 31/381, A61K 31/5375, A61P 25/28, A61P 43/00, C07D 265/30

(54) **AGENT FOR PROMOTING THE RECOVERY FROM DYSFUNCTION AFTER THE ONSET OF CENTRAL NEUROLOGICAL DISEASE**

(30) Priority: 14.07.2004 JP 2004207234
(71) Applicant: Astellas Pharma Inc., Tokyo 103-8411 (JP)
(72) Inventor: YATSUGI, Sachiko, Astellas Pharma Inc., Chuo-ku, Tokyo, 1038411 (JP); TAKAHASHI, Masayasu, Astellas Pharma Inc., Chuo-ku, Tokyo, 1038411 (JP); YATSUGI, Shinichi, Astellas Pharma Inc., Chuo-ku, Tokyo, 1038411 (JP)
(74) Representative: HOFFMANN EITLE
(86) International application number: PCT/JP2005/012895
(87) International publication number: WO 2006/006617

(57) **Abstract**

It is intended to provide a novel agent for promoting the recovery from dysfunction after the onset of a central neurological disease or an agent for enhancing and/or promoting the effect of rehabilitation for functional recovery. Namely, an agent for promoting the recovery from dysfunction after the onset of a central neurological disease which contains, as the active ingredient, a compound capable of simultaneously and selectively enhancing neurotransmission by serotonin and neurotransmission by norepinephrine; and an agent for enhancing and/or promoting the effect of rehabilitation for functional recovery after the onset of a central neurological disease which contains, as the active ingredient, a compound capable of simultaneously and selectively enhancing neurotransmission by serotonin and neurotransmission by norepinephrine. It is useful to provide the excellent agent for promoting the recovery from dysfunction after the onset of a central neurological disease and the agent for enhancing and/or promoting the effect of rehabilitation for functional recovery as described above. These drugs are also useful as safe drugs because of being free from anticholinergic effect causing side effects, drug dependence or effects on the circulatory organs.

## Description

### Technical Field

The present invention relates to a pharmaceutical drug, in particular, an agent for promoting the recovery from dysfunction after the onset of a central neurological disease and an agent for enhancing and/or promoting the effect of rehabilitation for functional recovery after the onset of a central neurological disease.

### Background Art

Many patients with central neurological disease, such as stroke, brain injury, spinal cord injury, and neurodegenerative disease are targets of rehabilitation medicine in Japan. These diseases include a variety of disorders: motor dysfunctions such as mobility disorders of the extremities and gait dysfunction, neurological disorders such as hemiplegia, psychiatric disorders such as cognitive impairment and depressive state, and the like. Drug therapy and rehabilitation for functional recovery are conducted, depending on the progress of a disease condition and the severity of disorder.
The ratio of stroke patients to the patients who are given rehabilitation medicine is highest. In Japan, stroke accounts for approximately 30% of the causes for becoming bedridden and the number of patients suffering from its sequela is estimated to be approximately 1.7 million (Guidelines for the Management of Stroke in Japan, 2004 (The Japan Stroke Society)). Particularly for motor dysfunction due to stroke, it is considered to be most effective to start rehabilitation for functional recovery at an early stage after the onset. The duration of rehabilitation is still long and its effect is not always sufficient, however. Accordingly, a drug that can further shorten the duration of rehabilitation for functional recovery and enhance the effect of rehabilitation to the maximum is required.
(S)-2-[[(7-Fluoro-4-indanyl)oxy]methyl]morpholine hydrochloride (referred to as Compound A hereinafter) was found as a compound having a serotonin reuptake inhibitory effect and a 5-HT2A receptor antagonism (Patent Document 1). In other words, it is reported that Compound A has the effect of enhancing serotonin neurotransmission based on the serotonin reuptake inhibitory activity as well as the effect of enhancing norepinephrine neurotransmission based on the 5-HT2A receptor antagonism, and its activity is comparable to that of venlafaxine, a serotonin-norepinephrine reuptake inhibitor, described below (Non-patent Document 1 and Non-patent Document 2). That is to say, Compound A is a compound that has the effect of enhancing both serotonin and norepinephrine neurotransmissions.

Patent Document 1 discloses that Compound A is useful as a therapeutic drug for secondary symptoms of a cerebrovascular disorder, such as a decrease of spontaneity and depressive mood, and as an agent for improving cerebral function due to its blood viscosity improving effect and antihypoxic effect. However, there is neither suggestion nor specific disclosure on the effect of promoting recovery from dysfunction, including motor dysfunction due to a central neurological disease, or the effect of enhancing and/or promoting the effect of rehabilitation for functional recovery.
It has been confirmed heretofore that enhancement of norepinephrine neurotransmission promotes the recovery from dysfunction after brain injury (Non-patent Document 3 and Non-patent Document 7), and the effect of D-amphetamine, a monoamine release promoter, has been studied well (Non-patent Document 4, Non-patent Document 5 and Non-patent Document 6). Fluoxetine, a selective serotonin reuptake inhibitor (SSRI), has also been reported to be effective for recovery of motor function (Non-patent Document 8).
Based on these findings, compounds having the effect of enhancing neurotransmission of the norepinephrine neurons or serotonin neurons are considered to be effective for promoting the recovery from dysfunction after the onset of a central neurological disease. However, D-amphetamine causes adverse drug reactions such as drug dependence, an excitatory effect, and effects on the circulatory system, and the effect of fluoxetine for promoting functional recovery is insufficient as compared with that of D-amphetamine (Non-patent Document 9). Accordingly, drugs that are safer and have excellent efficacy have been desired.

Patent Document 1: International Patent Publication WO94/18182 Pamphlet
Non-patent Document 1: Eur. J. Pharmacol. 395(1), 31-36, 2000
Non-patent Document 2: J. Pharmacol. Exp. Ther. 302 (3), 983-991, 2002
Non-patent Document 3: Science 217, 855-857,1982
Non-patent Document 4: Stroke 29, 2381-2395, 1998
Non-patent Document 5: Ann. Neurol. 23, 94-97, 1988
Non-patent Document 6: Stroke 26,2254-2259, 1995
Non-patent Document 7: Am. J. Phys. Med. Rehabil. 72, 286-293, 1993
Non-patent Document 8: Stroke 27, 1211-1214, 1996
Non-patent Document 9: Am. J. Phys. Med. Rehabil. 73, 76-83, 1994

### Disclosure of the Invention

### Problems to be Solved by the Invention

An object of the present invention is to provide a pharmaceutical drug useful as an agent for promoting the recovery from dysfunction after the onset of a central neurological disease and an agent for enhancing and/or promoting the effect of rehabilitation for functional recovery after the onset of a central neurological disease. Particularly, an object of the present invention is to provide a pharmaceutical drug superior to conventionally known drugs, fluoxetine, desipramine, and D-amphetamine, in terms of the above promotion and/or enhancement effects or reduction in adverse drug reactions.

### Means for Solving the Problems

The present inventors further studied drugs that promote the recovery from dysfunction due to a central neurological disease, and as the result confirmed that compounds that simultaneously and selectively enhance serotonin neurotransmission and norepinephrine neurotransmission, especially Compound A, have an excellent effect of promoting recovery of motor functions, and completed the present invention.
That is, the present invention relates to an agent for promoting the recovery from dysfunction after the onset of a central neurological disease such as stroke, brain injury, neurodegenerative disease, and spinal cord injury, comprising a compound that simultaneously and selectively enhances serotonin neurotransmission and norepinephrine neurotransmission as an active ingredient; and an agent for enhancing and/or promoting the effect of rehabilitation for functional recovery after the onset of a central neurological disease comprising a compound that simultaneously and selectively enhances serotonin neurotransmission and norepinephrine neurotransmission as an active ingredient.

### Effects of the Invention

The present invention is useful for providing an excellent agent for promoting the recovery from dysfunction due to a central neurological disease and an agent for enhancing and/or promoting rehabilitation for functional recovery. The pharmaceutical drug of the present invention is useful as a safe drug free from adverse drug reactions due to an anticholinergic effect, such as dry mouth, constipation, dysuria, and blurred vision, or drug dependence. Especially, Compound A is superior to conventionally known drugs, fluoxetine, desipramine, and D-amphetamine, in terms of the above promotion and/or enhancement effect or reduction in adverse drug reactions. In addition, Compound A has a protective effect on mitochondrial dysfunction and affinity to sigma receptors, thus has an inhibitory effect on cell death due to stroke and the effect of enhancing neurite outgrowth, and is useful as a therapeutic agent of the present invention.

### Best Mode for Carrying Out the Invention

The preferred aspects of the present invention will be described in the following items (1) to (8).
(1) An agent for promoting the recovery from dysfunction after the onset of a central neurological disease comprising a compound that simultaneously and selectively enhances serotonin neurotransmission and norepinephrine neurotransmission as an active ingredient.
(2) An agent for enhancing and/or promoting the effect of rehabilitation for functional recovery after the onset of a central neurological disease comprising a compound that simultaneously and selectively enhances serotonin neurotransmission and norepinephrine neurotransmission as an active ingredient.
(3) The agent described in item (1) or (2), wherein the central neurological disease is stroke, brain injury, spinal cord injury, or neurodegenerative disease.
(4) The agent described in item (3), wherein the dysfunction is motor dysfunction, sensory function disorder, or speech function disorder.
(5) An agent for promoting the recovery from dysfunction after the onset of a central neurological disease comprising duloxetine, venlafaxine, milnacipran, or (S)-2-[[(7-fluoro-4-indanyl)oxy]methyl]morpholine or a pharmaceutically acceptable salt thereof as an active ingredient.
(6) An agent for enhancing and/or promoting the effect of rehabilitation for functional recovery after the onset of a central neurological disease comprising duloxetine, venlafaxine, milnacipran, or (S)-2-[[(7-fluoro-4-indanyl)oxy]methyl]morpholine or a pharmaceutically acceptable salt thereof as an active ingredient.
(7) The agent described in item (5) or (6), wherein the central neurological disease is stroke, brain injury, spinal cord injury, or neurodegenerative disease.
(8) The agent described in item (7), wherein the dysfunction is motor dysfunction, sensory function disorder, or speech function disorder.
(9) An agent for promoting the recovery from dysfunction after the onset of a central neurological disease comprising a compound having a serotonin reuptake inhibitory effect and 5-HT2A receptor antagonism as an active ingredient.
(10) An agent for enhancing and/or promoting the effect of rehabilitation for functional recovery after the onset of a central neurological disease comprising a compound having a serotonin reuptake inhibitory effect and 5-HT2A receptor antagonism as an active ingredient.

(11) The agent described in item (9) or (10), wherein the central neurological disease is stroke, brain injury, spinal cord injury, or neurodegenerative disease.
(12) The agent described in item (11), wherein the dysfunction is motor dysfunction, sensory function disorder, or speech function disorder. The further preferable aspects of the present invention will be described in the following items (13) to (18).
(13) An agent for promoting the recovery from dysfunction after the onset of a central neurological disease comprising (S)-2-[[(7-fluoro-4-indanyl)oxy]methyl]morpholine or a pharmaceutically acceptable salt thereof as an active ingredient.
(14) An agent for enhancing and/or promoting the effect of rehabilitation for functional recovery after the onset of a central neurological disease comprising (S)-2-[[(7-fluoro-4-indanyl)oxy]methyl]morpholine or a pharmaceutically acceptable salt thereof as an active ingredient.
(15) The agent described in item (13) or (14), wherein the central neurological disease is stroke, brain injury, spinal cord injury, or neurodegenerative disease.
(16) The agent described in item (15), wherein the dysfunction is motor dysfunction, sensory function disorder, or speech function disorder.
(17) An agent for promoting the recovery from motor dysfunction after the onset of stroke comprising (S)-2-[[(7-fluoro-4-indanyl)oxy]methyl]morpholine or a pharmaceutically acceptable salt thereof as an active ingredient without causing accentuation of heart rate or excitatory effect.
(18) An agent enhancing and/or promoting the effect of rehabilitation for recovery from motor dysfunction after the onset of stroke comprising (S)-2-[[(7-fluoro-4-indanyl)oxy]methyl]morpholine or a pharmaceutically acceptable salt thereof as an active ingredient without causing accentuation of heart rate or excitatory effect.

The present invention will be described in more detail.
The "compound that simultaneously and selectively enhances serotonin neurotransmission and norepinephrine neurotransmission" means a compound that has an effect of simultaneously enhancing both serotonin neurotransmission and norepinephrine neurotransmission through uptake inhibition or release promotion of serotonin and norepinephrine. In other words, neither compounds that selectively enhance serotonin neurotransmission (for example, fluoxetine) nor compounds that selectively enhance norepinephrine neurotransmission (for example, desipramine) are included in the active ingredient of the present invention. The "compound that simultaneously and selectively enhances serotonin neurotransmission and norepinephrine neurotransmission" does not include compounds that enhance neurotransmission via dopamine, a kind of monoamine. In other words, conventionally known D-amphetamine is not included in the active ingredient of the present invention, since it promotes release of monoamines, but it is not a compound that simultaneously and selectively enhances serotonin neurotransmission and norepinephrine neurotransmission.

The active ingredient of the present invention includes, specifically SNRI and Compound A, and Compound A is particularly preferred.
The "SNRI" is a drug that has selective serotonin and norepinephrine uptake inhibitory effects, specifically including milnacipran (Asahi Kasei Corporation), venlafaxine (Wyeth), duloxetine (Lilly), F-98214-TA (FAES Farma), and the like. It should be noted that, although milnacipran, venlafaxine, and duloxetine are known as "antidepressants," they are not recognized as the agent of the present invention for promoting the recovery from dysfunction after the onset of a central neurological disease or an agent for enhancing and/or promoting the effect of rehabilitation for functional recovery after the onset of a central neurological disease.
The "dysfunction after the onset of a central neurological disease" means motor dysfunction, sensory function disorder, speech function disorder, and the like caused by disorder of nervous functions controlled by the sites damaged by stroke, brain injury, neurodegenerative diseases, spinal cord injury, and the like. It does not include psychiatric disturbance such as depressive symptom or cognitive impairment such as dementia. The dysfunction to which the present invention is preferably applied is motor dysfunction.
The "stroke" is classified into hemorrhagic and non-hemorrhagic. Examples of the hemorrhagic stroke include cerebral hemorrhage, subarachnoid hemorrhage, and intracranial hemorrhage secondary to cerebral arterial malformation, while examples of the non-hemorrhagic stroke include cerebral infarction.
The "brain injury" refers to a condition in which the brain is traumatically damaged by injury caused in a traffic accident or the like, including brain contusion, epidural hematoma, subdural hematoma, intracerebral hematoma, diffuse axonal injury, and the like.
The "spinal cord injury" refers to a condition in which the spinal cord receives compression/detrition due to a vertebral fracture or dislocation to cause dysfunction.
The "neurodegenerative disease" refers to a syndrome with chronic and progressive death of neuronal cells belonging to a particular functional system. Examples thereof include Parkinson disease, spinocerebellar degeneration, multiple system atrophy, amyotrophic lateral sclerosis, and the like.

The "motor dysfunction" refers to a condition with which performing voluntary movements is difficult, impossible, or not smooth, and it means motor paralysis and ataxia. Specifically it is disorder of skill movement, Babinski sign, spastic paralysis, spasticity (chronic phase), increased deep tendon reflex (chronic phase), muscle rigidity, bradypragia, involuntary movement (tremors, choreic movement, athetosis, dystonia, etc.), ataxia (extremities and trunk), speech function disorder, or eating disorder/dysphagia. The present invention is preferably applied to gait dysfunction and upper extremity function disorder.
The "sensory function disorder" refers to a condition in which cerebral disorder disables correct recognition of superficial sensation such as tactile sensation, baresthesia, and thermoesthesia; deep sensation such as position sensation and palleshesia; combined sensation such as two-point discrimination and graphesthesia; or the like. It is classified according to the severity into anesthesia (sensory extinction), hypesthesia (sensory blunting), hyperesthesia, and dysaesthesia (paresthesia). It also includes sensory function disorders characterized by the site of occurrence, such as dysethesia of half of the body, superficial sensation disorder, and whole sensation disorder.
The "speech function disorder" refers to aphasia characterized by decrease of ability in terms of language, such as listening, reading, speaking and writing words caused by a damage in the region controlling a language function; and dysarthria characterized by symptoms such as dysphonia and phonation disorder due to paralysis of the phonic and speech organs such as the lips, tongue and vocal band or disadaptation of movement (ataxia). The present invention is preferably applied to dysarthria due to motor dysfunction.
The "promotion of the recovery from dysfunction" means recovery from the above dysfunction earlier and to a higher level for purposes of shortening of a hospitalization period, and promoting early independence and improvement of quality of life (QOL), and the like after the onset.
The application of the present invention preferably relates to motor dysfunction due to stroke, brain injury, neurodegenerative disease, or spinal cord injury, more preferably to gait dysfunction and upper extremity dysfunction after stroke.
The "rehabilitation for functional recovery" means rehabilitation for recovery of motor functions, such as muscle-strengthening exercise, range-of-motion exercise of hand fingers, knees and the like, motion exercise such as walking; rehabilitation for recovery of language functions; rehabilitation for recovery of cognitive functions, and the like that are conducted in the acute phase, recovery phase, and maintenance phase according to the time after onset of a central neurological disease and the condition of a patient. The present invention is preferably applied to rehabilitation for recovery of motor functions.
The "enhancing the effect of rehabilitation for functional recovery" means recovery of a function to a higher level and further alleviation of a disorder as compared with the case of conducting rehabilitation alone.
The "promoting the effect of rehabilitation for functional recovery" means promotion of functional recovery in a shorter period as compared with that conducted by rehabilitation alone.

(S)-2-[[(7-Fluoro-4-indanyl)oxy]methyl]morpholine and its pharmaceutically acceptable salts can be readily obtained by the manufacturing method described in Patent Document 1 or manufacturing methods similar thereto.
Milnacipran can be readily obtained by the method described in US Patent US4478836, venlafaxine by the method described in British Patent No. GB2227743, duloxetine by the method described in US Patent US5362886, and F-98214-TA by the method described in Journal of Medicinal Chemistry (2003), 46(25), 5512-5532 or the methods similar thereto.
Compound A can form salts with acids besides hydrochloride. Such salts are included in the present invention as long as they are pharmaceutically acceptable salts. Specifically, examples of the acids include inorganic acids such as hydrobromic acid, hydroiodic acid, sulfuric acid, nitric acid, and phosphoric acid; and organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, and glutamic acid. Compound A includes various hydrates, solvates, and polymorphism of its free form and pharmaceutically acceptable salts.

The preparation of the present invention can be prepared using carriers for pharmaceuticals, excipients and the like usually used in the art by the methods usually used. Administration may be oral administration using tablets, pills, capsules, granules, powder, liquid, and the like, or parental administration using injections such as intraarticular, intravenous, intramuscular injections, suppository, eye drops, eye ointment, percutaneous liquid, ointment, percutaneous patch, transmucosal liquid, transmucosal patch, inhalant, and the like.
The form of the solid composition for oral administration according to the present invention includes tablet, powder, granule, and the like. For such a solid composition, one or more active ingredients are mixed with at least one inert diluent, for example, lactose, mannitol, glucose, hydroxypropylcellulose, microcrystalline cellulose, starch, polyvinylpyrrolidone, and/or magnesium aluminate metasilicate. The composition may contain, according to conventional manners, additives other than the inert diluents exemplified by lubricants such as magnesium stearate, disintegrants such as cellulose calcium gluconate, stabilizers, and solubilizing aids. Tablets and pills may be coated with sugar, such as sucrose, gelatin, hydroxypropylcellulose, hydroxypropylmethylcellulose phthalate and the like, or a film of a gastric or enteric substance.
The form of the liquid composition for oral administration includes pharmaceutically acceptable emulsion, solution, suspension, syrup, elixir, or the like, and the liquid composition contains a generally used inert diluent, for example, purified water or ethanol. The liquid composition may contain aids such as solubilizing agents, moisturizers, and suspending agents, sweeteners, flavors, aromatic substances, or preservatives in addition to the inert diluent.

The injection for parenteral administration contains a sterile aqueous or non-aqueous solvent, suspending medium, or emulsifying medium. The aqueous solvent or suspending medium includes distilled water for injection and physiological saline. The non-aqueous solvent or suspending medium is exemplified by propylene glycol, polyethylene glycol, vegetable oils such as olive oil, alcohols such as ethanol, Polysolvate 80 (the name in the Japanese Pharmacopeia), and the like. These compositions may further contain tonicity agents, preservatives, moisturizers, emulsifiers, dispersing agents, stabilizers, or solubilizing aids. These compositions are sterilized, for example, by filtration through a bacteria-retentive filter, by addition of a bactericide, or by irradiation. Alternatively, a sterile solid composition may be prepared and, prior to use, dissolved or suspended in sterile water or a sterile solvent for injection.
The transmucosal preparations such as a transnasal preparation may be solid, liquid or semi-liquid and can be manufactured according to conventionally known methods. For example, publicly known pH adjusters, preservatives, thickeners, and excipients may be added as appropriate and the composition is formed into solid, liquid or semi-liquid. The transnasal preparation is administered through an ordinary spray apparatus, a nasal spray, a tube, an insert for the nasal cavity, or the like.
The drug used in the present invention is administered to patients having dysfunction after the onset of a central neurological disease, preferably to patients immediately after to within 6 months after the onset, over the period of rehabilitation for functional recovery (1 month to 1 year at the maximum, preferably 3 to 6 months). An appropriate daily dose for oral administration is usually about 0.01 to 1000 mg, preferably 0.1 to 300 mg/kg, further preferably 0.1 to 100 mg, and the daily dose is administered once or divided into 2 to 4 portions daily over the period of rehabilitation for functional recovery. For intravenous administration, an appropriate daily dose is about 0.001 to 100 mg/kg body weight and the daily dose is administered once or divided into several portions daily. A dose may be determined individually according to symptoms, age, sex, and the like.

### Examples

The present invention will be illustrated in more detail referring to Examples.

### Example 1

### Evaluation Test for Walking Function in Cerebral Infarction Rat Model

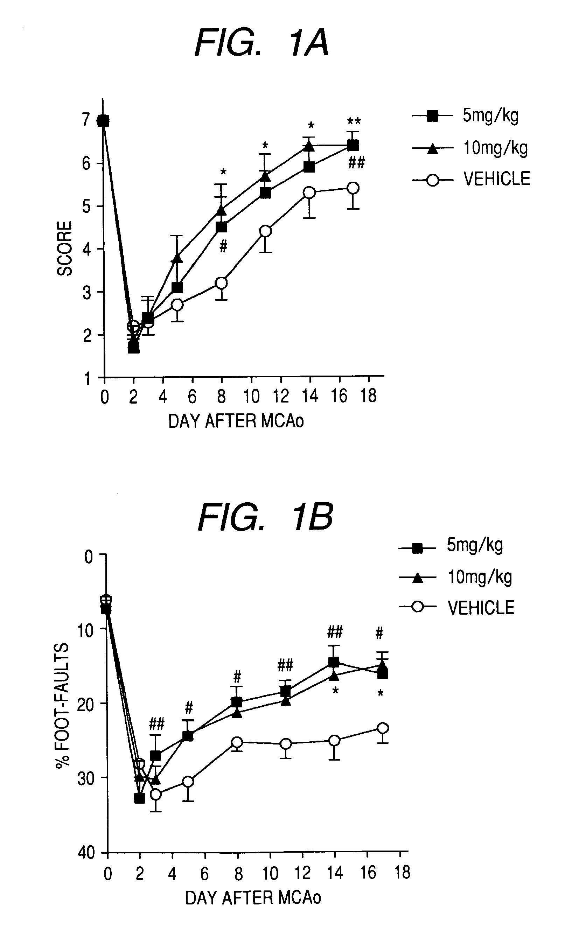

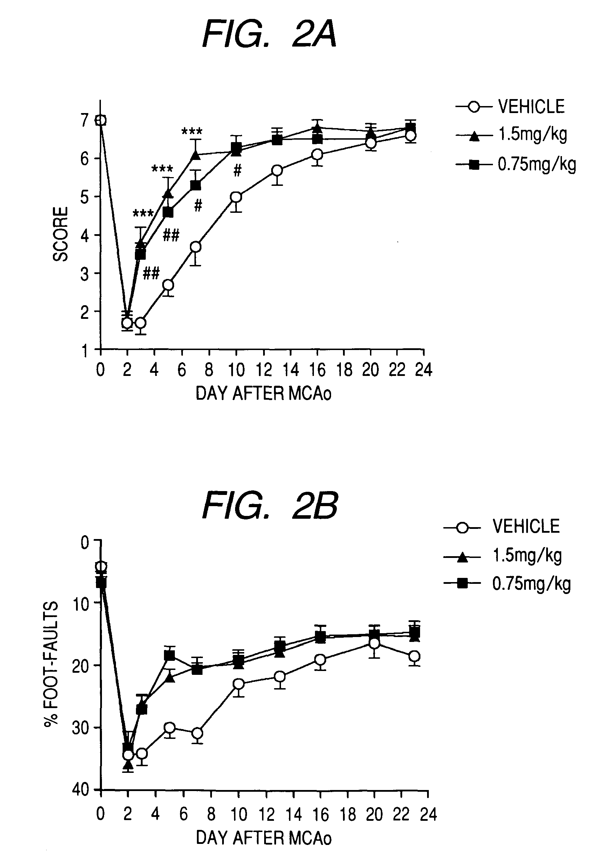

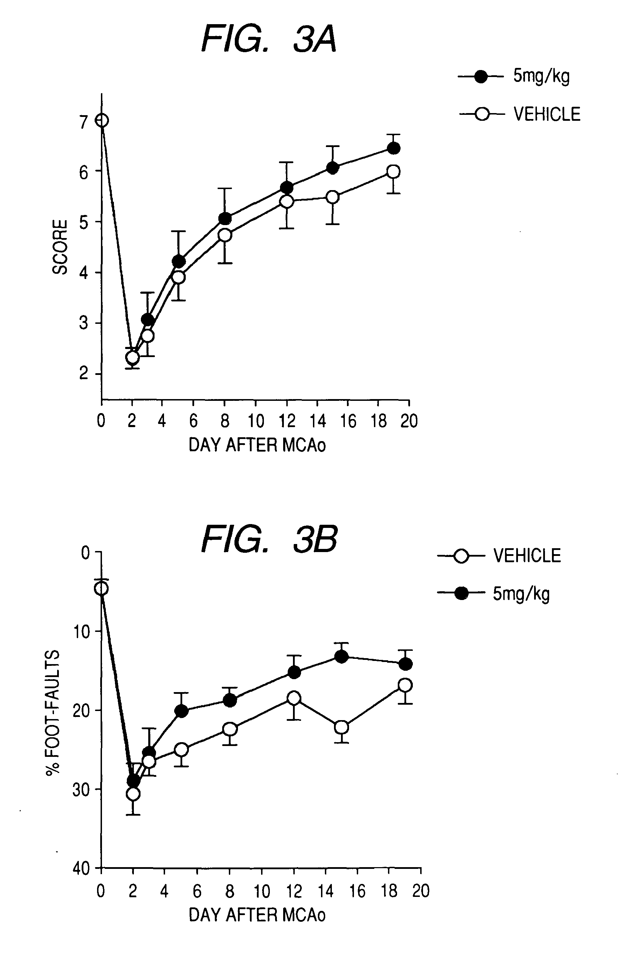

A cerebral infarction rat model was prepared according to the method described in J. Cereb. Blood Flow Metab. 8, 474-485, 1988. Male spontaneous hypertension rats (Hoshino Laboratory Animals) weighing 278 to 350 g at the time of operation were used and a cerebral infarction was prepared by obstructing the left common carotid artery and the left middle cerebral artery. The walking function was evaluated using a beam-walking test and a foot-fault test in accordance with the method described in J. Neurotrauma 13, 293-307, 1996. In the beam-walking test, walking ability during walking on a beam 18 mm in width and 122 cm in length was scored into 7 grades (7 corresponds to normal and 1 corresponds incapability of walking). In the foot-fault test, during walking on a grid of 53 cm x 36 cm with a lattice size of 6.5 mm² for 2 minutes, a percentage of the number of foot slips with respect to the total number of steps of the forepaw on the disordered side was measured. The tests were conducted immediately before preparation of cerebral infarction, 2, 3, and 5 days after preparation of cerebral infarction, and thereafter twice a week. Compound A and fluoxetine were administered after measurement of pre-drug values at 2 days after preparation of cerebral infarction and treatment was continued until the completion of evaluation. Compound A was administered 45 minutes prior to the walking function tests and fluoxetine was administered 60 minutes prior to the tests. Distilled water in the same volume was administered to the control group. D-amphetamine was administered intraperitoneally 60 minutes prior to the walking function tests from 3 days to 2 weeks after preparation of cerebral infarction. Physiological saline in the same volume was administered to the control group. From the observed values, the corresponding pre-drug values were subtracted, and statistical analysis were performed by two-way repeated-measures analysis of variance. When a significant difference was observed, a multiple comparison was conducted. The results are shown in Figs. 1 to 3.
As shown in Fig. 1, Compound A exhibited a significant improvement effect as compared with the control group in the beam-walking test (a) and foot-fault test (b) [Beam-walking test: two-way analysis of variance; p-value = 0.0096 for treatment effect, p-value <0.001 for time effect, p-value =0.021 for interaction. Multiple comparison; *: p<0.05, ** : p<0.01 (10 mg/kg vs. vehicle), #: p<0.05, ##: p<0.01 (5 mg/kg vs. vehicle). Foot-fault test; two-way analysis of variance; p-value =0.0019 for treatment effect, p-value <0.001 for time effect, p-value =0.022 for interaction. Multiple comparison; *: p<0.05.(10 mg/kg vs. vehicle), #: p<0.05, ##: p<0.01 (5 mg/kg vs. vehicle)).
As shown in Fig. 2, D-amphetamine exhibited a significant improvement effect as compared with the control group in the beam-walking test (a) [Two-way analysis of variance; p-value =0.0055 for treatment effect, p-value <0.001 for time effect, p-value <0.001 for interaction. Multiple comparison; ***:p<0.001 (1.5 mg/kg vs. vehicle), #: p<0.05,##: p<0.01 (0.75 mg/kg vs. vehicle)). However, in the foot-fault test, although a tendency toward improvement was observed as compared with the control group, the effect was not significant.
As shown in Fig. 3, fluoxetine exhibited no evident improvement effect as compared with the control group in the beam-walking test (a) and foot-fault test (b).
Accordingly, the above tests show that Compound A has a promoting effect on recovery from gait function disorder caused by cerebral infarction superior to D-amphetamine and fluoxetine.

### Example 2

### Evaluation Test for Forepaw Function in Cerebral Infarction Rat Model (Staircase Test)

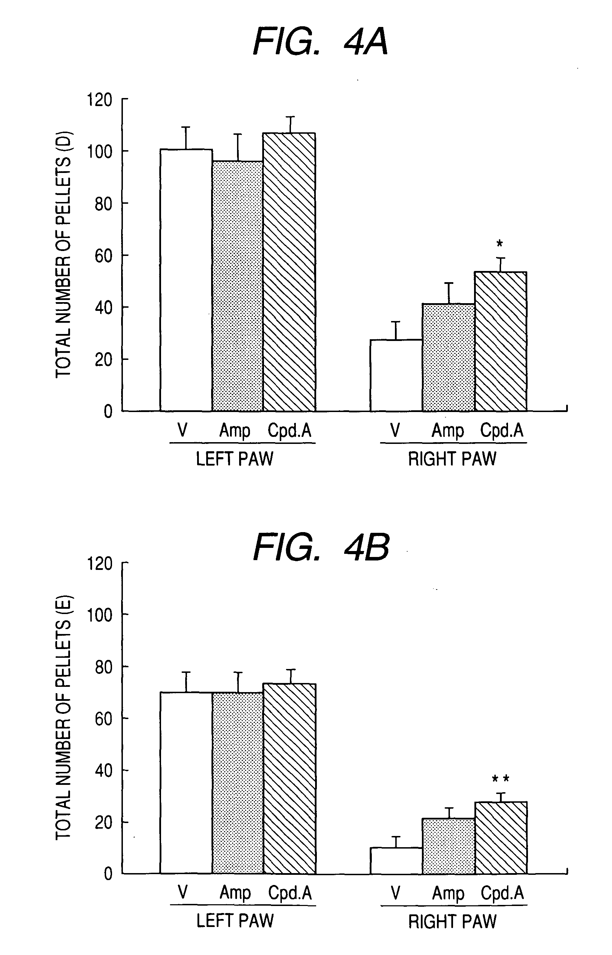

Male spontaneous hypertension rats weighing 251 to 339 g (Hoshino Laboratory Animals) were used. The forepaw function was evaluated using a staircase test (a test using a case in which a base was placed and stairs were set in parallel on both left and right sides of the base within an acrylic box having a size to accommodate one rat) in accordance with the method described in J. Neurosci. Methods 36, 219-228, 1991. A rat for which feed had been limited prior to the experiment was placed in a staircase and trained to acquire the skill of eating food pellets set on a dent on each stair step prior to counting. The numbers of pellets displaced and the numbers of pellets eaten were counted for the right and left forepaws. After acquisition of the eating skill, cerebral infarction was prepared as in Example 1. Compound A was administered orally at a dose of 5 mg/kg five times per week from the next week following cerebral infarction preparation to week 6. D-amphetamine was administered intraperitoneally at a dose of 1.5 mg/kg twice per week from the next week following cerebral infarction preparation to week 6. Distilled water or physiological saline in the same amount was administered to the control group. The test was conducted 60 minutes after drug administration. Drug efficacy was analyzed by the Dunnett's multiple comparison. The results are shown in Fig. 4.
As shown in Fig. 4, the forepaw dysfunction is compared by using the number of displaced pellets (a) and the number of eaten pellets (b) in the staircase test as indices, indicating that a significant improvement effect was observed in the Compound A group as compared with the control group (*:p<0.05, **: p<0.01 vs. vehicle). Although a tendency toward improvement was observed in the D-amphetamine group as compared with the control group, the effect was not significant.
Accordingly, the above test shows that Compound A has a promoting effect on recovery from forepaw dysfunction caused by stroke superior to that of D-amphetamine.

### Example 3

### Evaluation of Effect on locomotor activity

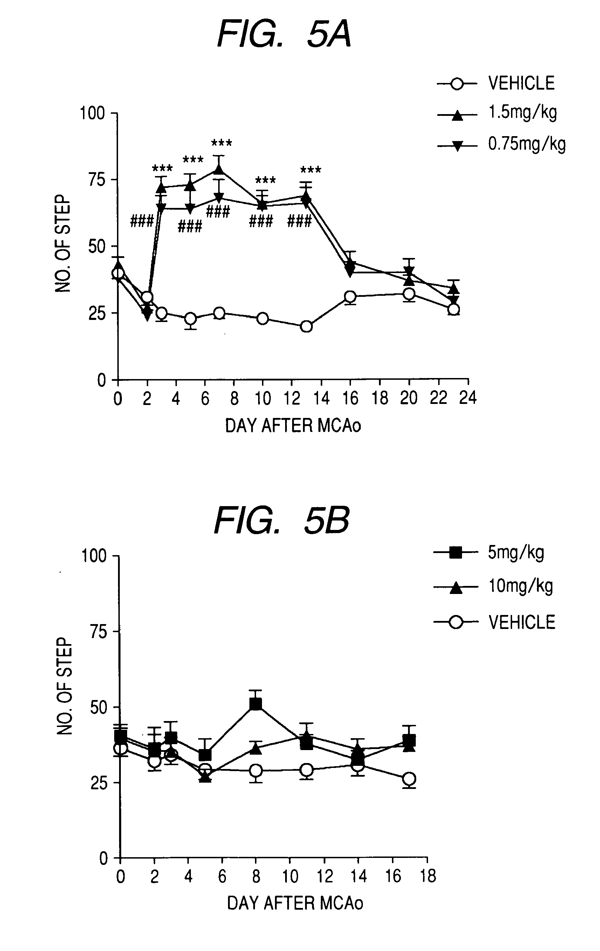

The effects of D-amphetamine and Compound A on the locomotor activity were evaluated using the number of total steps in the foot-fault test in Example 1. Statistical analysis was conducted by two-way repeated-measures analysis of valiance. When a significant difference was observed, a Dunnett's multiple comparison was conducted. The results are shown in Fig. 5.
As shown in Fig. 5, an excitatory effect was developed and a remarkable increase in the number of steps was observed in the above test during the administration period in the D-amphetamine group (a) [Two-way analysis of variance; p-value <0.001 for treatment effect, p-value <0.001 for time effect, p-value <0.001 for interaction.
Multiple comparison; ***: p< 0.001 (1.5 mg/kg vs. vehicle), ###: p<0.001 (0.75 mg/kg vs. vehicle)]. On the other hand, no effect on the number of steps was observed in the Compound A group (b).
Accordingly, it is revealed that D-amphetamine exhibits simultaneously an unfavorable excitatory effect, at the dose exhibiting drug efficacy on the functional recovery, whereas Compound A does not have such an unfavorable effect.

### Example 4

### Evaluation of Effect on the Circulatory System

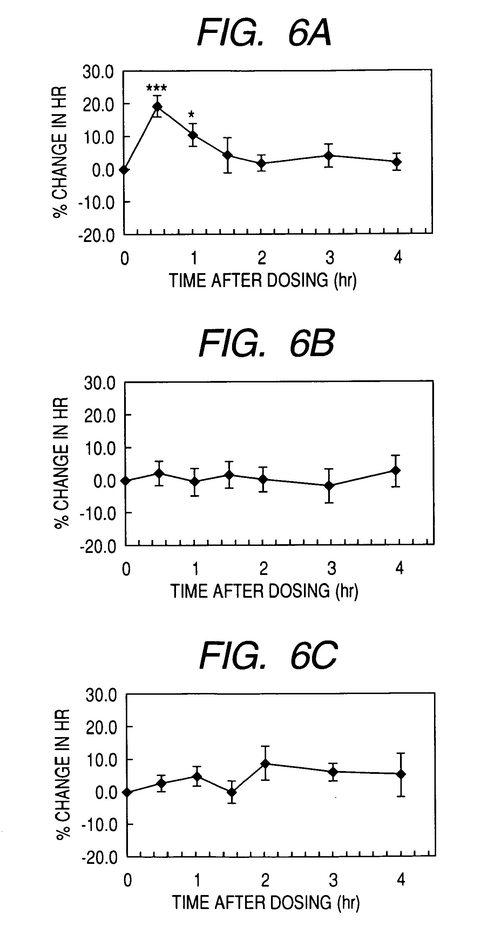

Male spontaneous hypertension rats (Hoshino Laboratory Animals) weighing 240 to 275 g were used. After a cannula was inserted into the common carotid artery, heart rates were monitored. Compound A or D-amphetamine was administered orally or intraperitoneally, respectively, and the heart rates were recorded until 4 hours after administration. For the control group, physiological saline was administered intraperitoneally and the measurement was similarly conducted. Statistical analysis was conducted by one-way repeated-measures analysis of variance. When a significant difference was observed, a multiple comparison by the two-way Dunnet method was applied. The results are shown in Fig. 6.
As shown in Fig. 6, a significant accentuation of heart rate was observed from 30 minutes to 1 hour after administration in the D-amphetamine group (a) (One-way analysis of variance: p-value <0.001, Multiple comparison; ***: p<0.001, *: p<0.05 vs. basal value). On the other hand, no effect on heart rate was observed in the Compound A group (b) or the control group (c).
Accordingly, it is revealed that D-amphetamine also exhibits an unfavorable effect, an accentuation of heart rate (circulatory effect), at the dose exhibiting drug efficacy on the functional recovery, whereas Compound A does not have such an unfavorable effect.

In conclusion, concerning Compound A and fluoxetine, as shown in Figs. 1 to 3, Compound A exhibited an improvement effect better than that of fluoxetine in the beam-walking test and the foot-fault test. Accordingly, Compound A is more useful than fluoxetine as an agent for promoting the recovery from dysfunction after the onset of a central neurological disease and an agent for enhancing and/or promoting the effect of rehabilitation for functional recovery after the onset of a central neurological disease.
Concerning Compound A and D-amphetamine, as shown in Figs. 1, 2, and 4, it is found that Compound A has drug efficacy equal to or better than D-amphetamine in the beam-walking test, the foot-fault test, and the staircase test. In addition, it is found from Figs. 5 and 6 that D-amphetamine also exhibits unfavorable side effects such as excitatory effect and an accentuation of heart rate at the dose exhibiting the same efficacy. Accordingly, Compound A is an agent for promoting the recovery from dysfunction after the onset of a central neurological disease and an agent for enhancing and/or promoting the effect of rehabilitation for functional recovery after the onset of a central neurological disease, which has a wider safety range than D-amphetamine and has no adverse drug reaction at the dose exhibiting drug efficacy.

### Brief Description of Drawings

[Fig.1] Fig. 1 shows the improving effect of Compound A on gait function in the beam walking test (a) and the foot-fault test (b). (Example 1) (In the figure, the"score" represents scores of the beam-walking test, the "day after MCAo" represents the days after preparation of cerebral infarction, the "% foot-faults" represents a percentage of the number of foot slips with respect to the total number of steps in foot-fault tests, and the "vehicle" represents the control group receiving solvent)
[Fig. 2] Fig. 2 shows the effect of D-amphetamine on gait function in the beam-walking test (a) and the foot-fault test (b). (Example 1) (The symbols in the figure are the same as above.)
[Fig. 3] Fig. 3 shows the effect of fluoxetine on gait function in the beam-walking test (a) and the foot-fault test (b). (Example 1) (The symbols in the figure are the same as above.)

[Fig. 4] Fig. 4 shows the effect of Compound A and D-amphetamine on the forepaw function with the number of displaced food pellets (a) and the number of eaten pellets (b) as indices. (Example 2). (In the figure, the "Total Number of Pellets (D)" represents the number of diplaced pellets, the "Total Number of Pellets (E)" represents the number of eaten pellets, the "Left Paw" represents a forepaw on the healthy side, the "Right Paw" represents the forepaw on the disordered side, the "V" represents the control group receiving solvent, the "Amp" represents the D-amphetamine group, and the "Cpd.A" represents the Compound A group.)
[Fig. 5] Fig. 5 shows the changes in the number of steps in the D-amphetamine group (a) and the Compound A group (b). (Example 3) (In the figure, the "No. of step" represents the number of steps in the foot-fault test, and the other symbols are the same as those in Fig. 1).
[Fig. 6] Fig. 6 shows the changes in heart rate in the D-amphetamine group (a), Compound A group (b), and control group (c). (Example 4) (In the figure, the "% Change in HR" represents the rate of changes in the heart rate, and the "time after dosing (hr)" represents time after administration.)

### Industrial Applicability

As described above, the present invention can be applied as a drug, particularly an agent for promoting the recovery from dysfunction after the onset of a central neurological disease and an agent for enhancing and/or promoting the effect of rehabilitation for functional recovery after the onset of a central neurological disease.

## Claims

1. An agent for promoting the recovery from dysfunction after the onset of a central neurological disease comprising a compound that simultaneously and selectively enhances serotonin neurotransmission and norepinephrine neurotransmission as an active ingredient.

2. An agent for enhancing and/or promoting the effect of rehabilitation for functional recovery after the onset of a central neurological disease comprising a compound that simultaneously and selectively enhances serotonin neurotransmission and norepinephrine neurotransmission as an active ingredient.

3. The agent according to claim 1 or 2, wherein the central neurological disease is stroke, brain injury, spinal cord injury, or neurodegenerative disease.

4. The agent according to claim 3, wherein the dysfunction is motor dysfunction, sensory function disorder, or speech function disorder.

5. An agent for promoting the recovery from dysfunction after the onset of a central neurological disease comprising duloxetine, venlafaxine, milnacipran, or (S)-2-[[(7-fluoro-4-indanyl)oxy]methyl]morpholine or a pharmaceutically acceptable salt thereof as an active ingredient.

6. An agent for enhancing and/or promoting the effect of rehabilitation for functional recovery after the onset of a central neurological disease comprising duloxetine, venlafaxine, milnacipran, or (S)-2-[[(7-fluoro-4-indanyl)oxy]methyl]morpholine or a pharmaceutically acceptable salt thereof as an active ingredient.

7. The agent according to claim 5 or 6, wherein the central neurological disease is stroke, brain injury, spinal cord injury, or neurodegenerative disease.

8. The agent according to claim 7, wherein the dysfunction is motor dysfunction, sensory function disorder, or speech function disorder.

9. An agent for promoting the recovery from dysfunction after the onset of a central neurological disease comprising a compound having a serotonin reuptake inhibitory effect and 5-HT2A receptor antagonism as an active ingredient.

10. An agent for enhancing and/or promoting the effect of rehabilitation for functional recovery after the onset of a central neurological disease comprising a compound having a serotonin reuptake inhibitory effect and 5-HT2A receptor antagonism as an active ingredient.

11. The agent according to claim 9 or 10, wherein the central neurological disease is stroke, brain injury, spinal cord injury, or neurodegenerative disease.

12. The agent according to claim 11, wherein the dysfunction is motor dysfunction, sensory function disorder, or speech function disorder.

13. An agent for promoting the recovery from dysfunction after the onset of a central neurological disease comprising (S)-2-[[(7-fluoro-4-indanyl)oxy]methyl]morpholine or a pharmaceutically acceptable salt thereof as an active ingredient.

14. An agent for enhancing and/or promoting the effect of rehabilitation for functional recovery after the onset of a central neurological disease comprising (S)-2-[[(7-fluoro-4-indanyl)oxy]methyl]morpholine or a pharmaceutically acceptable salt thereof as an active ingredient.

15. The agent according to claim 13 or 14, wherein the central neurological disease is stroke, brain injury, spinal cord injury, or neurodegenerative disease.

16. The agent according to claim 15, wherein the dysfunction is motor dysfunction, sensory function disorder, or speech function disorder.

17. An agent for promoting the recovery from motor dysfunction after the onset of stroke comprising (S)-2-[[(7-fluoro-4-indanyl)oxy]methyl]morpholine or a pharmaceutically acceptable salt thereof as an active ingredient without causing accentuation of heart rate or excitatory effect.

18. An agent enhancing and/or promoting the effect of rehabilitation for recovery from motor dysfunction after the onset of stroke comprising (S)-2-[[(7-fluoro-4-indanyl)oxy]methyl]morpholine or a pharmaceutically acceptable salt thereof as an active ingredient without causing accentuation of heart rate or excitatory effect.
